(19) Europäisches Patentamt
European Patent Office
Office européen des brevets

(11) **EP 3 108 833 A1**

(12) **EUROPEAN PATENT APPLICATION**

(43) Date of publication:
**28.12.2016 Bulletin 2016/52**

(51) Int Cl.:
***A61B 17/56*** (2006.01)

(21) Application number: **15305975.3**

(22) Date of filing: **24.06.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicants:
• **Université de Strasbourg**
  **67000 Strasbourg (FR)**
• **CENTRE NATIONAL DE**
  **LA RECHERCHE SCIENTIFIQUE -CNRS-**
  **75794 Paris Cedex 16 (FR)**
• **Institut National Des Sciences**
  **Appliquees**
  **67000 Strasbourg (FR)**

(72) Inventors:
• **LEPOUTRE, Nicole**
  **67100 STRASBOURG (FR)**
• **MEYLHEUC, Laurence**
  **67130 BERGBIETEN (FR)**
• **BARA, Gabriela Iuliana**
  **67400 ILLKIRCH (FR)**
• **BAYLE, Bernard**
  **67000 STRASBOURG (FR)**

(74) Representative: **Novagraaf Technologies**
  **Bâtiment O2**
  **2, rue Sarah Bernhardt**
  **CS90017**
  **92665 Asnières-sur-Seine Cedex (FR)**

(54) **METHOD FOR CONTROLLING THE VISCOSITY OF ORTHOPEDIC BONE CEMENT**

(57) The object of the invention is a method for controlling the viscosity of orthopedic bone cement during its curing in percutaneous vertebroplasty that prevents both aforementioned drawbacks, notably by allowing a controlled heating and/or cooling of said cement during the injection that leads to a dynamic and full control of the viscosity of the cement during the injection.

FIG. 3

EP 3 108 833 A1

**Description**

**TECHNICAL FIELD OF THE INVENTION**

The present invention refers to a method for controlling the viscosity of orthopedic bone cement during its curing in percutaneous vertebroplasty. The present invention is also related to an injection device that allows said control.

Percutaneous vertebroplasty is a non-surgical minimal invasive intervention that involves injecting bone cement into the vertebral body of a patient, under medical imaging control, to reinforce or restructure a weakened or broken vertebra. Such an intervention allows the stabilization of the vertebrae and the reduction of severe pain for people suffering from vertebral compression fractures, most often caused by osteoporosis but also, less frequently, by metastases or traumatic fractures[1].

During a vertebroplasty intervention, patients are generally placed in prone position under conscious sedation (mild sedation and analgesia). Depending on the size of each pathological vertebrae and its level of inside damage, the practitioner inserts one or two bone trocars via a transpedicular approach using Computed Tomography (CT) or fluoroscopic guidance. Once the trocars inserted, the orthopedic bone cement is typically prepared (mixing phase) by mixing a powder based on polymethylmethacrylate (PMMA) and a liquid form of methylmethacrylate (MMA). To promote the exothermic free radical polymerization process, the powder also incorporates an initiator while the liquid includes an activator. In order to obtain a radio-opaque cement, a radiopacifier figures also in the powder. The mixing phase ends when the cement is homogeneous. Then, a waiting phase occurs until its viscosity achieves a minimum threshold, which is left to the decision of the physician. Indeed, the operator draws on his experience to know when the cement is ready to be injected, which is a highly subjective diagnosis. This phase can last one or two minutes. Afterwards, the radiologist fills the conventional syringe or the delivery device and plugs it to the inserted needle. The cement injection occurs at that time under continuous radioscopic control in order to identify any potential leaks. This working phase (or hardening phase) does not last more than 10 to 15 minutes since afterwards the cement becomes too viscous to be injected.

Despite fast and significant benefits from the patient's perspective, this procedure introduces two high-risk sources:

- firstly, defined as a postoperative complication, the risk of cement leakage outside the damaged vertebra is critical since the consequences might be dramatic. Indeed, the patient may end up with a pulmonary embolism due to cement leakage. This risk is all the more significant given that the bone cement has a low viscosity at the beginning of the injection;
- secondly, the practitioner's permanent exposure to harmful X-rays causes adverse effects on his health.

The aim of this invention is therefore to remedy to the first above-mentioned drawbacks, notably to avoid or at least to reduce this risk of cement leakage. In that context, the Applicant has now developed a method for controlling the viscosity of orthopedic bone cement during its curing by acting on the bone cement temperature in percutaneous vertebroplasty. The Applicant has also developed an injection device (not claimed) for implementing said control during a vertebroplasty intervention, to remedy to the second above-mentioned drawback.

The skilled person knows methods involving the cooling or the heating of the cement during the intervention. However, in these known methods, the cooling of the cement is limited to a pre- or per-operative conservation of its fluidity before the actual injection[2], [3].

Furthermore, it is also known by the man skilled in the art to use heating of the cement to increase its viscosity[4] but the heating is not dynamically controlled. At last, even if the injection device of US 8,523,871[4] implements both heating and cooling functions, only the actual heating can be controlled because of the positioning of the sensor.

**SUMMARY OF THE INVENTION**

The invention therefore proposes a method for controlling the viscosity of orthopedic bone cement during its curing in percutaneous vertebroplasty that prevents both aforementioned drawbacks, notably by allowing a controlled heating and/or cooling of said cement during the injection that leads to a dynamic and full control of the viscosity of the cement during the injection.

In the method taught by US patent US 8,523,871[4], the principle consists in using a slow curing bone cement and to manage its viscosity at the entrance of the vertebra via a radiofrequency energy. As a result, leakage risks consistently decrease. However, the drawbacks of such a method are that the physician has to know how to use RF pulse and this method is cement-dependent. At last, temperatures inside the vertebra may reach values up to 200°C, which adds possible complications towards tissue neighboring the damaged vertebra.

Unlike the method taught by US 8,523,871[4], the method of the invention allows a precise, dynamic and full control of the temperature of the cement in a given section of the pipe in order to follow a viscosity set point $\eta^*$, evolving

over time in a given interval $[\eta_{min}, \eta_{max}]$. The control of the bone cement temperature consists in:

- accelerating the curing reaction by heating if the viscosity of the cement is lower than $\eta^*$;
- slowing down the curing reaction by cooling if the viscosity of the cement is higher than $\eta^*$ (in US 8,523,871[4], even if both heating and cooling functions are implemented, only the heating can be controlled).

The present invention relates to a method for the dynamic control of the viscosity of orthopedic bone cement during its curing by acting on the bone cement temperature in percutaneous vertebroplasty, within an injection device comprising a syringe, a percutaneous needle connected to the syringe via a pipe, comprising an active heat exchanger.

According to the invention, the method comprises the following steps:

A. defining the time $t_o$, time at which the radiologist starts the mixing process of the bone cement;

B. filling the syringe with the prepared bone cement;

C. defining for said bone cement a target viscosity $\eta^*$ to be reached or maintained, said target viscosity $\eta^*$ being comprised in the range $[\eta_{min} - \eta_{max}]$, $\eta_{min}$ being the minimal threshold viscosity of the cement which has to be reached for beginning the injection and $\eta_{max}$ being the maximum threshold viscosity of the cement above which the injection is not possible anymore;

D. beginning the injection of the bone cement into the vertebra;

E. at instant t during the injection:

e1) measuring the effective temperature T of the bone cement at the outlet of the active heat exchanger and, possibly, measuring the effective temperature $T_i$ of the bone cement at its inlet;

e2) computing the pressure drop $\Delta P = P_o - P_i$ along the pipe between the outlet of the syringe and a given intermediate point, $P_o$ being the pressure measured at the outlet of the syringe and $P_i$ being the pressure measured at the given intermediate point on the pipe, the length between those two points being denoted as $L_{sensor}$;

e3) computing the flow rate Q of the bone cement in the pipe;

e4) computing the shear rate $\dot{\gamma}_p$ at the wall of the pipe as a function of the flow rate Q, the cross-section dimensions of the pipe and the intrinsic physical parameters of the cement;

e5) calculating the instant viscosity:

- $\eta(t, \dot{\gamma}_P, T)$ if Q is nonzero, as a function of time t, temperature T, flow rate Q, pressure drop $\Delta P$ and shear rate $\dot{\gamma}_p$;
- $\eta_0(t, T)$ if Q has a zero value, as a function of time t and temperature T;

e6) computing a set point temperature $T^*(t)$ associated to the target viscosity $\eta^*$ and the effective viscosity $\eta$, $\eta^*$ being function of the flow rate Q and the time t;

e7) calculating the difference $\varepsilon_T$ between the previously determined set point temperature $T^*(t)$ and the effective temperature at the outlet of the heat exchanger T;

e8) controlling the cooling or the heating of the bone cement throughout the control of the active heat exchanger as a function of $\varepsilon_T$;

F. at instant $t+\Delta t$, redefining possibly the set point viscosity $\eta^*$ in the range $[\eta_{min}; \eta_{max}]$ and repeating step E until the end of the injection, unless $\eta_0(t,T)$ has reached the maximum threshold viscosity $\eta_{max}$.

In the method of the invention, the first step consists in defining the time $t_o$, at which the radiologist starts the mixing process of the bone cement (step A). As indicated above, the mixing phase typically consists in mixing a powder based on polymethylmethacrylate (PMMA), an initiator and a radiopacifier, and a liquid form of methylmethacrylate (MMA) and an activator.

The mixing phase ends when homogeneous cement is reached. Afterwards, the radiologist fills the syringe, places it on the delivery device (not claimed) and plugs it to the inserted needle (step B).

Once the delivery device is ready for use, the cement injection (step D) occurs under continuous radioscopic control in order to identify any potential leaks.

In the normal course, the working phase does not last more than 10 to 15 minutes since afterwards the cement becomes too viscous to be injected and has reached a maximum threshold viscosity $\eta_{max}$ above which the injection is not possible anymore. Moreover, as explained above, the risk of leakage outside the damaged vertebra is considerable since bone cement has a very low viscosity at the beginning of the injection. This brief stage leaves little time to the radiologist to perform his intervention.

Therefore, the objective of the method of the invention for controlling the viscosity of the bone cement during its curing is twofold:

- on the one hand, to accelerate the polymerization process until the bone cement reaches a viscosity $\eta_{min}$ that is the minimal threshold viscosity of the cement which has to be reached for beginning the injection, and
- on the other hand, to minimize the viscosity evolution rate to increase the working phase.

Once the viscosity passes this threshold $\eta_{min}$, the leakage risk is already highly reduced.

This lower viscosity limit is obtained by a preliminary study with the help of experimented radiologists. For this study, a physician prepares the bone cement. A small sample is collected and placed on the rotational rheometer while the rest of the mixture is poured out in the conventional injector. The expert is asked to inject slowly at a constant velocity so that the same shear rate can be applied on the sample on the dynamic rheometer. Once he estimates the minimum $\eta_{min}$ is attained, the viscosity value is read on the rheometer. Statistics on several trials with different physicians offer a good estimate $\eta_{min}$. This can be done for all the cements on the market.

The method of the invention necessarily comprises a step C to define, for the bone cement, a target viscosity $\eta^*$ to be reached or maintained, that is comprised in the range $[\eta_{min}\text{-}\eta_{max}]$, $\eta_{min}$ being the minimal threshold viscosity of the cement which has to be reached for beginning the injection and $\eta_{max}$ being the maximum threshold viscosity of the cement above which the injection is not possible anymore.

At instant t during the injection, the following measurements are made:

- the effective temperature T of the bone cement (step e1) at the outlet of the active heat exchanger, and possibly, the effective temperature $T_i$ of the bone cement at its inlet, and
- the pressure $P_o$ measured at the outlet of the syringe and the pressure $P_i$ at a given intermediate point on the pipe, thus giving the pressure drop $\Delta P = P_o\text{-}P_i$ along the pipe between the outlet of the syringe and the given intermediate point (step e2).

According to a first implementation of the method of the invention, step e2) may be realized between the outlet of the syringe and the outlet of the needle.

According to a second implementation of the method of the invention, step e2 may be realized between the outlet of the syringe and the outlet of the active heat exchanger.

In that case, the intravertebral pressure $P_{vertebra}$ may be computed according to formula (1):

$$(1)$$

$$P_{vertebra} = P_o \left( 1 - \frac{L_{vertebra}}{L_{sensor}} \right) + \frac{L_{vertebra}}{L_{sensor}} P_i$$

with:

- $L_{vertebra}$ being the length comprised between the outlet of the syringe and the outlet of the needle.

Advantageously, the step of computing the flow rate Q of the bone cement in the pipe may comprise a step of measuring a moving speed $V_{pist}$ of the piston of the syringe, said piston being driven to vary the volume of the cement in the syringe, the volumetric flow Q being then given by $Q = V_{pist}.\pi.r^2$, where r is the radius of the pipe.

At instant t during the injection, besides the above-mentioned measurements the following calculations are also made:

- the flow rate Q of the bone cement in the pipe (step e3)
- the shear rate $\dot{\gamma}_p$ at the wall of the pipe as a function of the flow rate Q, the cross-section dimensions of the pipe and the intrinsic physical parameters of the cement (step e4),
- the instant viscosity (step e5) $\eta(t,T,\dot{\gamma}_P)$ if Q is nonzero and or $\eta_0(t,T)$ if Q has a zero value,
- the set point temperature $T^*(t)$ associated to the target viscosity $\eta^*$ (step e6), and
- the difference $\varepsilon_T$ between the previously determined set point temperature $T^*(t)$ and the effective temperature at the outlet of the heat exchanger T (e7).

The instant viscosity $\eta(t,T,\dot{\gamma}_P)$, if the flow rate is nonzero, may be calculated according to modified Power Law as defined by formula (2) in the case of a pipe having a cylindrical geometry of radius r:

$$(2) \quad \eta(t, T, \dot{\gamma}_P) = a_{T_0}(T)K(t)\left(a_{T_0}(T)\dot{\gamma}_P\right)^{n(t)-1}$$

with

$$a_{T_0}(T) = \exp\left(\frac{-E_a}{R}\left(\frac{1}{T} - \frac{1}{T_0}\right)\right)$$

with:

- $E_a$ being the activation energy in $J.mol^{-1}$,
- T being the effective temperature of the bone cement at the outlet of the active heat exchanger,
- To being a reference temperature at which the viscosity $\eta_0$ is known,
- R being the gas constant,
- n(t) being the flow index of the bone cement at the current time t, n is either a known constant or defined as a function of t.
- $\dot{\gamma}_p$ being the shear rate at the wall of the pipe being given by formula (3):

$$(3) \qquad \dot{\gamma}_P = \frac{Q}{\pi r^3} \frac{3n(t) + 1}{n(t)}$$

with r being the radius of the pipe.
- K(t) being given by formula (4):

$$(4) \qquad Q = \left(\frac{\Delta P}{L_{sensor}}\right)^{1/n(t)} \left(\frac{r}{2K(t)}\right)^{1/n(t)} \left(\frac{\pi n(t) r^3}{3n(t) + 1}\right)$$

Advantageously, the instant viscosity $\eta_0(t,T)$, whether for a flow rate being nonzero or having a zero value, may be calculated according to the differential equation (5) :

$$(5) \qquad \dot{\eta}(t, T, \dot{\gamma}_P) = f(\eta(t, T, \dot{\gamma}_P))$$

where the time derivative $\dot{\eta}$ of the viscosity is defined as a function the viscosity $\eta$.

At the beginning of the injection, in order to reach as soon as possible the lower limit $\eta_{min}$, it is preferable to heat the PMMA. According to the results of an off-line characterization of bone cements, the higher the temperature of the cement is, the faster its viscosity increases. However, a runaway reaction is not excluded if the cement is warmed too much, so caution is a watchword. At the same time, reducing the injection flow can shorten the waiting time, since at low shear rates viscosity is higher.

As regards to the generation of the set point temperature T*, the most sensitive part of the invention lies in the injection time that has to be extended. On the opposite, the colder the cement stays, the longer the curing reaction lasts. Hence, to increase the injection time, it is preferable to cool the bone cement. Note that it is impossible to completely stop the increase of viscosity. Its evolution can just be slowed down. This leads to the set point T* temperature.

Preferably, minimizing PMMA viscosity evolution can be expressed as:

$$min \ (\dot{\eta}(t, T, \dot{\gamma}_P))$$

Thus, the set point temperature T*(t) may be calculated via

$$\underset{T}{argmin} \ \dot{\eta}$$

According to the method of the invention, the control (step e8) of the cooling or the heating of the bone cement is realized throughout the control of the active heat exchanger as a function of $\varepsilon_T$.

Preferably, the controlling e8) of the active heat exchanger may achieve the cooling or heating of the bone cement as a function of $\varepsilon_T$ throughout a temperature regulation scheme composed of two nested closed-loops, where:

- a temperature controller $C_T$ uses the difference $\varepsilon_T$ between the previously determined set point temperature T*(t) and the effective temperature T to compute the current reference I* of the active heat exchanger, the current reference I* being limited by a current saturation block,
- a current controller $C_I$ uses the difference $\varepsilon_I$ between the current reference I* and the effective input current I to compute the input voltage U of a power supply H driving the active heat exchanger.

At instant t+$\Delta$t, the set point viscosity $\eta$* may be redefined in the range [$\eta_{min}$; $\eta_{max}$] and step E is repeated until the end of the injection, unless $\eta_0$(t,T) has reached the maximum threshold viscosity $\eta_{max}$.

The present invention also relates to an injection device of curing cement for percutaneous vertebroplasty, said device comprising a system for generating volumetric flow of said cement, and a pipe connecting said injection device to a percutaneous needle, said injection device being characterized in that it further comprises at least one active heat exchanger(s) located on the pipe for dynamic controlled heating and/or cooling of said cement during the injection.

By active heat exchanger, it is meant according to the present invention, a heat exchanger that operates in cooling or heating with external energy and controls the heat exchanges.

Contrary to the teaching of US patent US 8,523,871[4] (where even if both heating and cooling functions are implemented, only the heating can be controlled), the active heat exchanger of the injection device of the invention allows a precise, dynamic and full control of the temperature of the cement in a given section of the pipe in order to follow a viscosity set point $\eta$*, evolving over time in a given interval [$\eta_{min}$, $\eta_{max}$]. This control is achieved by:

- accelerating the curing reaction by heating if the viscosity of the cement is lower than $\eta$*;
- slowing down the curing reaction by cooling if the viscosity of the cement is higher than $\eta$*.

As the cement itself acts as a thermal insulator, it is possible to regulate it on reduced pipe diameters and therefore it is less convenient to do it on the syringe as claimed in the US patent application US2013/0190680[2].

The system for generating volumetric flow of the device of the present invention may advantageously comprise a syringe for containing the cement, and a piston, that can move inside the syringe for pushing the cement inside the pipe through the syringe outlet.

According to a realization of the invention, the said active heat exchanger(s) located along the pipe may comprise a thermal block with at least one Peltier module mounted on the pipe. Preferably, the thermal block may comprise:

- a central regulated block made out of a thermal conducting material with low thermal inertia,
- at least one stack on the regulated block and including:

  • a Peltier module,
  • at least one heat sink made out of thermal conducting material with low thermal inertia,
  • a fan,

- a thermal insulation wrapping the central block, to improve the efficiency of the heat exchanger, and
- at least one temperature sensor.

According to another embodiment of the invention, the injection device of the invention may also further comprise a deported active heat exchanger put on a closed fluid circuit with a fluid-to-cement heat exchanger. The deported active heat exchanger of the invention operates in cooling and/or in heating, in order to reduce the exchanger's footprint at the proximity of the patient.

Preferably, the deported active heat exchanger of the invention may also comprise at least one Peltier module.

Advantageously, in this deported embodiment, the heat transfer fluid flowing between the deported active heat exchanger and the fluid-to-cement exchanger may be a liquid, a gas or a mixture of liquid and gas, and preferably water.

Besides the active heat exchanger(s) located on the pipe, the injection device of the invention may also advantageously further comprise at least one heat exchanger on the syringe, notably to cool the cement contained inside the syringe before being injected in the vertebra.

Advantageously, the heat exchanger on the syringe may be a passive heat exchanger, in order to keep the physical properties of the cement as constant as possible through the injection when not circulating in the pipe. Preferably, the passive heat exchanger located on the syringe may comprise a sheath surrounding the syringe that is preferably filled with a eutectic fluid such as a gel.

Advantageously, the said active and/or passive heat exchangers may be removable.

Advantageously, the injection device may also comprise a pressure sensor presenting a sensing area, said pressure sensor being located on a defined point of the cement pipe. Preferably, the sensing area of said pressure sensor may not be in direct contact with the cement.

Advantageously, the pressure of the cement in the pipe may be transmitted to the sensing area of said pressure sensor by an intermediary incompressible material, preferably water or an elastomeric material. Preferably, the cement pipe further may further comprise a sterile elastomeric membrane, able to transmit the cement pressure to said pressure sensor.

The injection device of the invention presents the major advantages:

- to give the physician an enhanced control over the injection procedure,
- to give the physician a pretty complete information concerning the cement and the injection state, that allows the injection of the cement at a high viscosity in order to reduce the leakage risk, and
- to optimize the cement working period.
- to remotely monitor intra vertebral pressure.


## BRIEF DESCRIPTION OF THE DRAWINGS

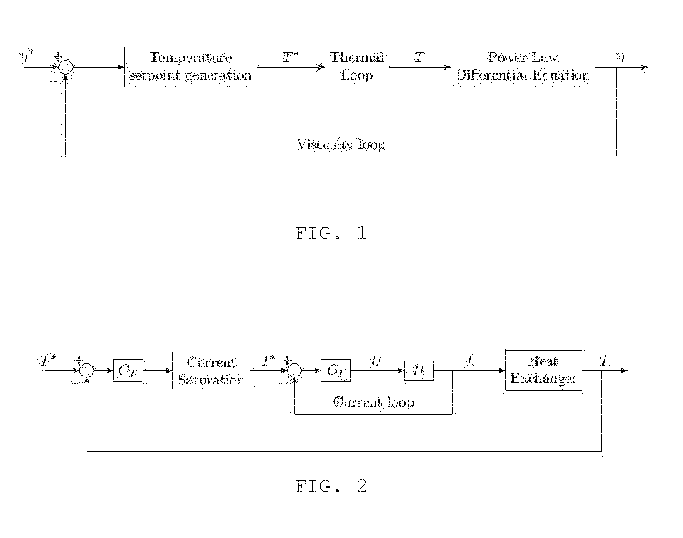

Other features and advantages of the invention will become more clearly apparent on reading the following description, given with reference to the appended figures, which illustrate non-limiting examples of preferred realizations (FIGS. 1 and 2) and also non limiting examples of different realizations of an injection device that may be used in the method of the invention:

- FIG. 1 represents schematically the viscosity regulation scheme composed of three nested closed-loops for realizing the controlling e8) of the active heat exchanger of an injection device, according to a preferred embodiment of the method of the invention,
- FIG. 2 represents schematically the thermal loop that is nested in the viscosity loop illustrated on FIG. 1,
- FIG. 3 represents a tridimensional CAD general view of an injection device that is used in the method of the invention as a whole,
- FIG. 4 represents a schematic diagram of the injection device illustrated on figure 3,
- FIG. 5 represents a CAD view of a master device that remotely controls the injection,
- FIG. 6A represents a tridimensional CAD view of a heat exchanger with a thermal block,
- FIG. 6B represents its corresponding cross-sectional schematic view,
- FIG. 7 represents a principle diagram of a deported active heat exchanger put on a closed fluid circuit with a fluid-to-cement heat exchanger, according to an embodiment of the invention,
- FIG. 8 represents a detailed view of the deported active heat exchanger illustrated on FIG. 7,
- FIG. 9 represents a detailed view of the water-to-cement heat exchanger illustrated on FIG. 7,
- FIG. 10 represents an exploded view of a sheath surrounding the syringe, according to an realization of the invention,
- FIG. 11 represents a detailed view of the syringe with sheath illustrated on FIG. 10,
- FIG. 12 represents a cross-sectional schematic view of an embodiment of a pressure sensor (located on the cement pipe) with a water-filled channel,
- FIG. 13 represents a cross-sectional schematic view of another embodiment of a pressure sensor with an elastomer-filled channel,
- FIG. 14 represents a cross-sectional schematic view of another embodiment of a pressure sensor with both an elastomer-filled channel and an elastomeric membrane.

For the sake of clarity, identical or similar elements have been referenced with identical reference symbols in all the figures.

**DETAILED DESCRIPTION OF THE PREFERRED EMBODIMENTS**

For purposes of understanding the principles of the invention, reference will now be made to the realizations illustrated in the drawings and the accompanying text.

The objective of the method of the invention is to control the cement viscosity. A regulation scheme composed of three nested closed-loops may be used for realizing the controlling e8) of the active heat exchanger of an injection device, as shown by FIGS. 1 and 2:

A. after defining for the bone cement to be injected the target viscosity $\eta^*$ to be reached or maintained, said target viscosity $\eta^*$ being comprised in the range [$\eta_{min}$-$\eta_{max}$], $\eta_{min}$ being the minimal threshold viscosity of the cement which has to be reached for beginning the injection and $\eta_{max}$ being the maximum threshold viscosity of the cement above which the injection is not possible anymore;

B. the set point temperature T*(t) associated to the target viscosity $\eta^*$ is computed according to the method of the invention (step e6) in the *"temperature set point generation block"* in the viscosity loop of FIG. 1,

C. the value of the set point temperature T*(t) is injected in the temperature regulation loop that is illustrated on FIG. 2, in which a temperature controller $C_T$ uses the difference $\varepsilon_T$ between the previously determined set point temperature T*(t) and the effective measured temperature T to compute the current reference I* of the active heat exchanger (always using electrical energy as input),

D. the current reference I* is limited by a current saturation block,

E. the current is also controlled in a closed loop control (current loop), in which a current controller $C_I$ uses the difference $\varepsilon_I$ between the current reference I* and the effective input current I to compute the input voltage U of a power supply H driving the active heat exchanger (current loop).

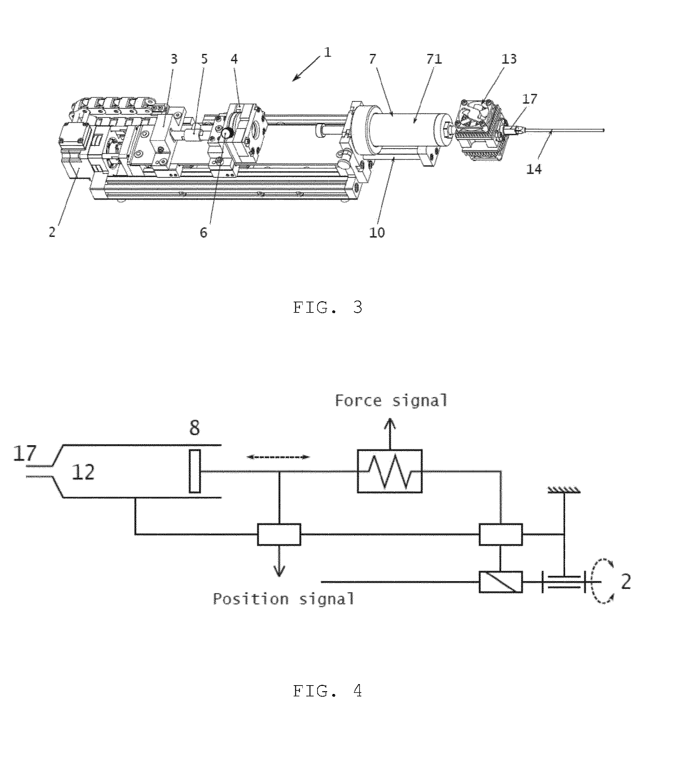

Now concerning the injection device 1 that may be used in the method of the invention, FIG. 3 represents a tridimensional (3D) CAD general view of the entire injection device 1 of the invention as a whole. It is based on a quite straightforward design, using a ball screw linear axis to transform the rotation of the rotor of a high torque servo-motor 2 into a linear translation, in order to push on a high-pressure syringe 7. Two separated mobile carts are placed on the axis, with the first one (3) being motorized by the screw while the second one (4) is capable of moving along the axis freely. The two carts 3 and 4 are linked together through a force sensor 5 in order to measure the linear force running through the assembly 1. A hand maneuvered clamping device 6 has been placed on the free cart 4. It is used to grip a specific syringe piston rod 8 and has been equipped with a low force limit switch to provide an automatic approach during the setup phase. An incremental encoder has also been added between the free cart 4 and the base in order to provide a direct reading of the cart position without being affected by the potential backlash and the flexibility of the kinematic chain.

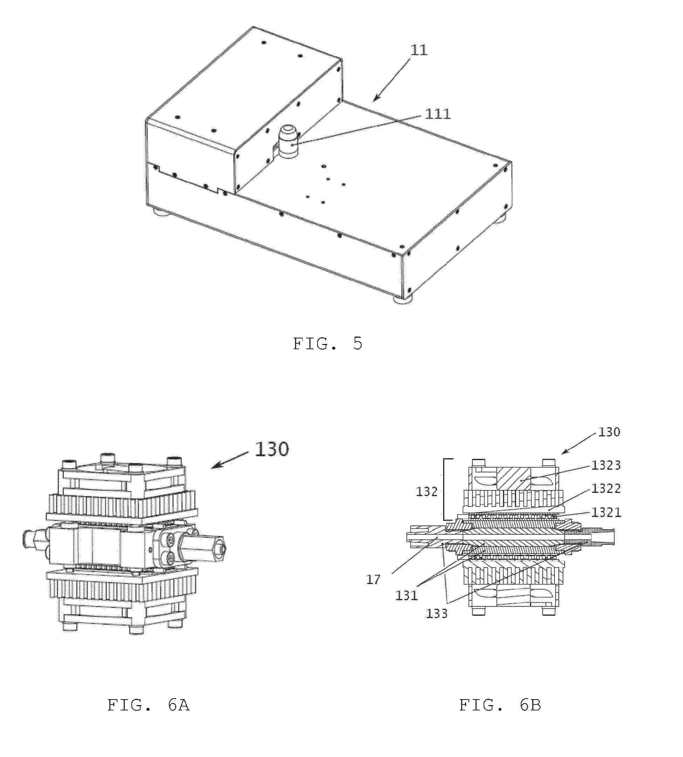

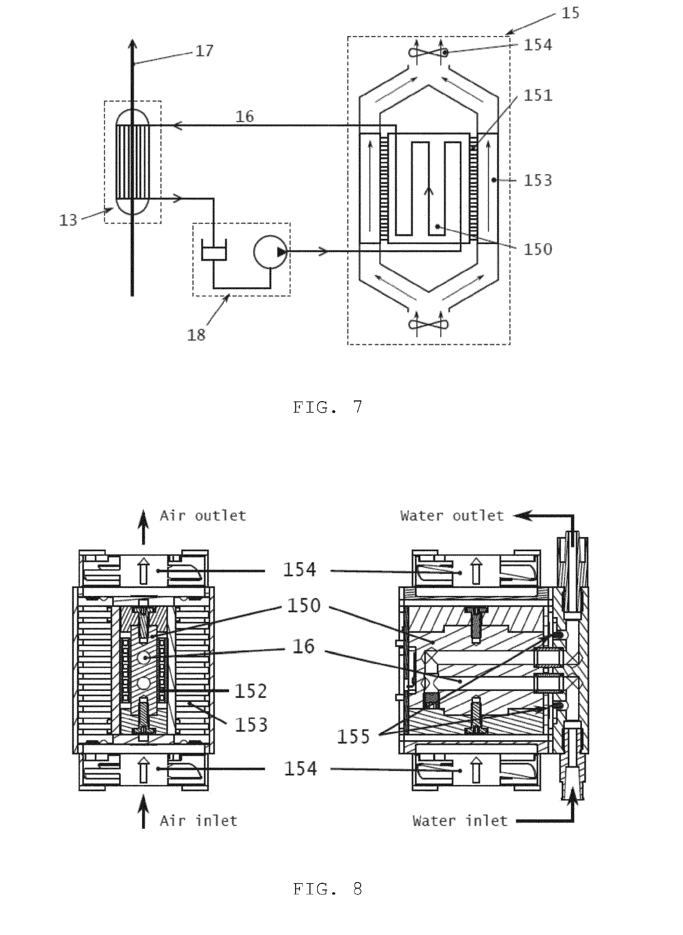

On the fixed part of the device, a mounting base has also been designed to support the syringe. The syringe itself is fixed to the mounting base by using a specific sheath that will be more precisely illustrated and detailed below (see FIGS. 6 and 7 and the corresponding accompanying paragraphs of the text). The syringe 7 is disposable because of the medical nature of its use.

FIG.3 also shows a percutaneous needle 14 connected to the syringe 7 via a pipe 17. An active heat exchanger 13 such as a thermal block (see also figures 4A and 4B) is located on the pipe 17) (surrounding said pipe 17) for the dynamic controlled heating and/or cooling of the cement 12 during the injection.

FIG. 4 represents a schematic diagram of the injection device shown on FIG. 3. The motor input 2 provide the displacement of the piston 8 that pushes the cement 12 in the cement channel 17. As shown of the diagram, both the position and force signal are provided by the system.

The overall dimensions of the injection device 1 as a whole are approximately 500 x 100 x 100 mm for a mass of approximately 5.5 kg. It can provide a service load of 2kN. This can generate a pressure of about 100 bar on the cement 12 in the syringe 7, and will allow to inject a fluid with a viscosity up to 2000 Pa.s at a flow rate of 33 mm$^3$/s considering the pressure drop of a 150 x Ø 2.5 mm cylinder (equivalent to a typical cement near the end of its solidification injected in a typical large section injection needle plugged into a short channel).

A master device 11, illustrated on FIG. 5, controls the injection remotely. In order to give to the physician the same feedback as he would have in a manual injection, the master device 11 can provide the force feedback of the pressure applied to the cement 12. Concerning the control of the injection itself, it is a flow rate control, which is more precise and practical than a volumetric control, generally provided by the current known manual systems. It then adds a design constraint to the master device 11 that should return to neutral position when the physician releases the interface because of safety issues. Besides that, the remote control also allows some flexibility as it may have some scaling or non-linear fitting both on the force-feedback and on the control signals, giving the possibility to customize easily both features with the experienced feedback of the practitioner.

The control of the injection is done via a rotating knob 111 located on the master device that returns the pressure information in the form of a force feedback. Should the physician release the knob during the injection, an integrated spring returns the interface in a neutral position.

As regards the active thermal regulation, which is realized in the method the invention by an active heat exchanger 13, FIGS. 6A and 6B show a first embodiment of an active heat exchanger 13 consisting in a unique thermal block 130. A thermal insulation (not shown on these figures) wraps the central part of the block 130. The thermal block is composed of

- a central regulated block 131 that is crossed by the cement pipe 17.
- two stacks 132, disposed symmetrically to the regulated block which include :

  - two Peltier modules 1321,
  - two heat sinks 1322,
  - two fans 1323,

- a thermal insulation (not shown on figures 4A and 4B) wrapping the central block 131, which reduces the thermal exchanges between the regulated volume and the ambient air,
- three temperature sensors (not shown on figures 6A and 6B) placed respectively on the central block 131 and on both heat sinks 1322 to measure the temperature of the regulated part and to control both Peltier modules, and
- two Luer-lock connections at the extremity of the channel 17 to plug the thermal block 130 to the syringe 7 on one side and to the needle 14 on the other side

The active thermal regulation may also be alternatively realized by a deported active heating/cooling heat exchanger 15 put on a closed water circuit 16 with a water-to-cement heat exchanger 13 located on the pipe 17 of the injection device 1, according to a second realization of the invention. Such a deported device allows a remote control of the viscosity of the cement during the intervention, thus protecting the radiologist during the cement injection phase by keeping her/him outside the radiation area.

FIG. 7 represents a schematic diagram of this closed water circuit 16 comprising the deported heat exchanger 15 and the water-to-cement heat exchanger 13 of the injection device 1. The water circulation is powered by a tanking/pumping device 18 placed on the closed loop 16. The advantage of this thermal regulation, in comparison with the thermal block of the first realization, is the possibility of doing the same regulation at a remote location, as the thermal block 130 of the first embodiment is both fragile, heavy and space consuming in a critical place such as the surgical area.

The deported heat exchanger 15 of FIGS. 7 and 8 (detailed section of Fig 7) comprises:

a heat transfer block 150 being crossed by a water circuit 16 connected to the water-to-cement heat exchanger 13,

- Peltier cells 152
- heat sinks 153, and
- fans 154.
- temperature sensors 155 placed on the water circuit 16.

As the orientation of the fan/sink couple has an impact on the performance on the heat sinks dissipation in free airflow, the fans 154 have been placed in a geometry designed to provide a more efficient forced airflow.

The water circuit 16 shown on FIG.8 also comprises a pumping system 18 that is able to work in reverse direction in order to purge the circuit 16 and thus, to avoid water leakage when the physician unplugs the exchanger 13 from the circuit 16.

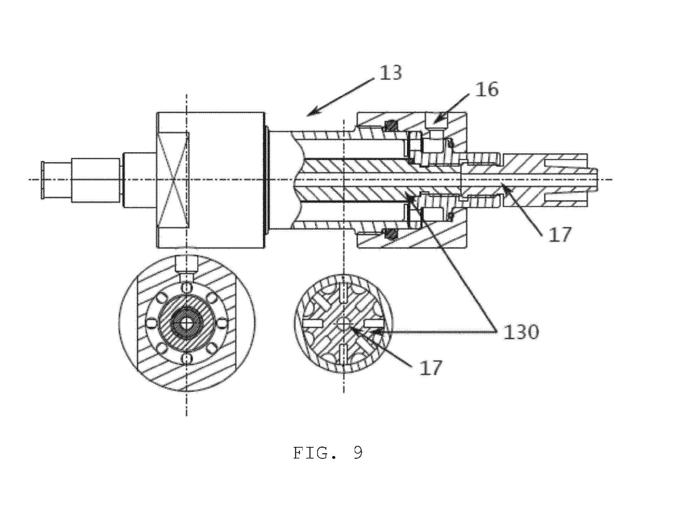

The water-to-cement heat exchanger 13 shown on FIG. 9 is built around a finned block 130 whose task is to ensure a proper heat transfer between the cement pipe 17 and the water circuit 16. In this realization, it replaces the thermal block 13 presented in FIG. 1 on the cement pipe 17.

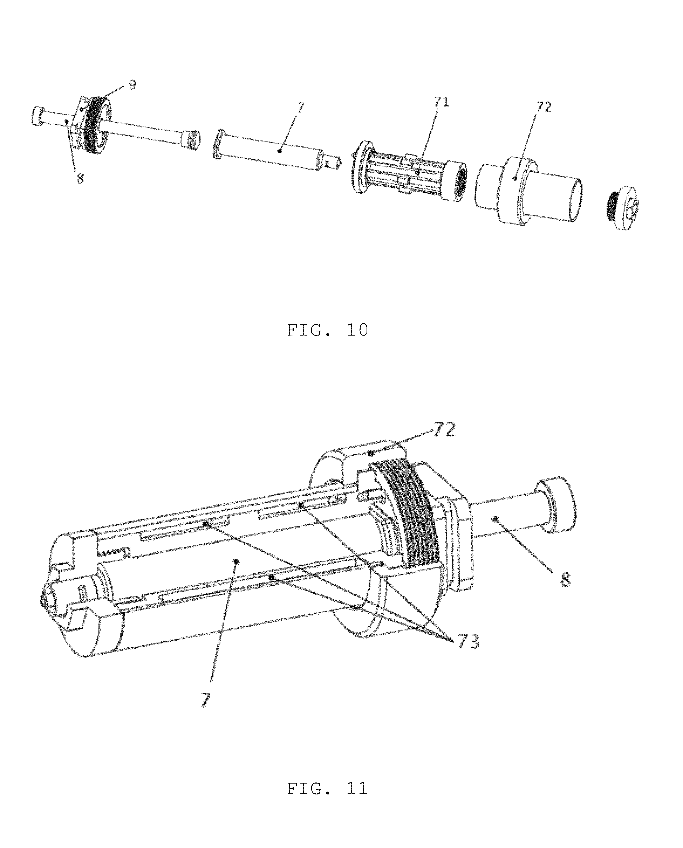

Now concerning the passive thermal exchange, FIGS. 10 and 11 represent a sheath 71 surrounding the syringe 7, according to an realization of the invention. In order to design this sheath, several constraints were taken in account:

- it has to resist the service load that may rise up to 2kN,
- it should be able to passively exchange thermal energy as to maintain the cement stored within the syringe 7 with the lowest viscosity possible throughout the injection,
- it has to be easily interfaced to the injection device 1 by having a dedicated interface 9 and by allowing a fast and easy locking of the syringe piston 8 to the free cart 4.

As such, the sheath 71 has been machined out of a 316L stainless steel in order to provide a high mechanical resistance and to resist to various chemical products, including biologic fluids and asepsis solutions. The sheath 71 provides some space around the syringe that may filled with an eutectic mixture known for its ability to exchange heat at constant temperatures, thus ensuring that the syringe 71 is kept cool during most of the injection.

The assembly (sheath) is also equipped with a fixation 9 at the back that interfaces with the mounting base 10 on the injector. A nut 72 and screw system is used to put in and extract the disposable syringe that contains the cement.

FIG 10 shows the syringe 71, the syringe sheath 71 (partly disassembled) and the high-pressure piston 8, while FIG. 11 shows a more detailed cutout of the sheath with the syringe 7 in it, where the room 73 for the eutectic gel is visible.

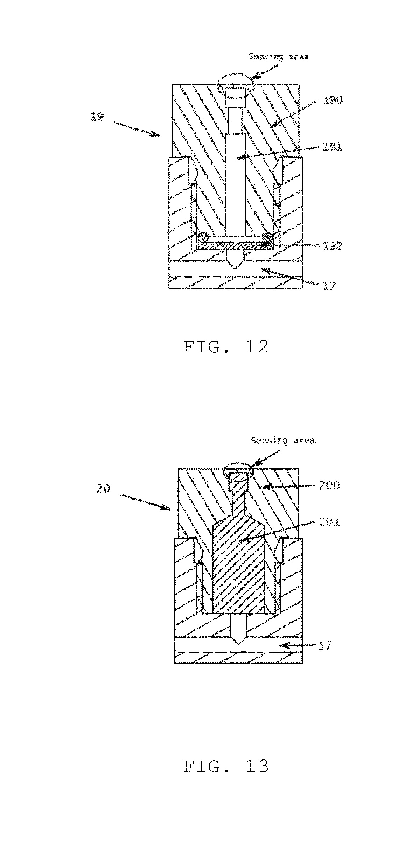

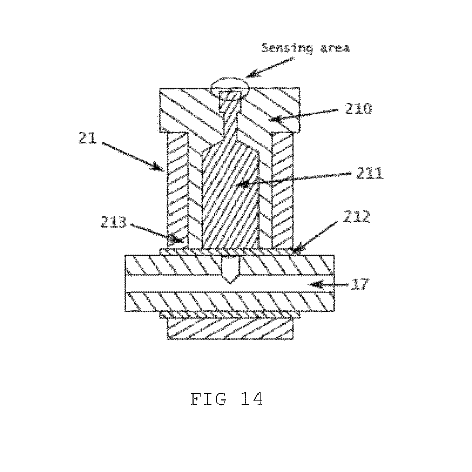

Now concerning pressure measurements of the cement along the cement pipe 17, FIGS. 12 to 14 are cross-sectional schematic views of different embodiments of pressure sensors 19, 20, 21 located on the pipe 17. In order to have a better control on and understanding of the injection procedure, it may be helpful to estimate the intravertebral pressure. This pressure may be used intraoperatively to detect failure during the procedure, such as pressure spikes or drops that may be symptomatic of clogging or leakage.

The best way to measure the intravertebral pressure would be to integrate a pressure sensor at the tip of the needle, but it would be very constraining in terms of design. Thus, in the frame of the present invention, the value of this pressure is obtained indirectly, using a pressure measurement in the cement channel.

Considering the flow of a cement with varying rheological parameters K and n in a cylindrical pipe of length $L_{vertebra}$ and of radius r, and given the known sensor position distant from the pipe inlet by a distance $L_{sensor}$, the Poiseuille flow leads to equation (6):

$$(6) \qquad Q = \left(\frac{\Delta P}{L_{sensor}}\right)^{1/n(t)} \left(\frac{r}{2K(t)}\right)^{1/n(t)} \left(\frac{\pi n(t) r^3}{3n(t)+1}\right)$$

Assuming that the flow rate is high enough, the cement viscosity at the sensor is substantially equal to the cement at the outlet of the pipe. This provide then equation (7):

$$(7) \qquad P_{vertebra} = P_o \left(1 - \frac{L_{vertebra}}{L_{sensor}}\right) + \frac{L_{vertebra}}{L_{sensor}} P_i$$

As shown by the equations, the knowledge of at least one pressure outside the injection pressure is mandatory. However, as pressure sensors are too expensive to be integrated as a disposable component, there is a need for a reusable pressure sensor that could be carried over several interventions. Also because of sterility issues, the sensor should not have any internal interface with the cement in order to be cleanable.

For this purpose, reusable pressure sensors have been developed in the frame of the present invention, in which the sensing area of a standard pressure sensor is immersed in an incompressible fluid that would transfer the pressure.

According to a first advantageous embodiment of such a pressure sensor 19 (as shown on FIG.10), it is based on a standard pressure sensor 190 whose channel 191 is filled with water. A flexible interface 192 separates the cement channel 17 and the sensor channel 191. The interface 192 may consist for instance in a cap made out of a flexible silicon compound, such a polydimethylsiloxane (PDMS).

According to a second advantageous embodiment of such a pressure sensor 20 (as shown on FIG.11), it is based on a standard pressure sensor 200 whose channel is filled with an elastomeric compound 201 such as PDMS.

According to a third advantageous embodiment of such a pressure sensor 21 (as shown on FIG.12), it is based on a standard pressure sensor 210 whose channel is filled with an elastomeric compound 211 such as PDMS. The cement pipe 17 is equipped with an elastomeric membrane 212, forming a measure point. The pressure sensor is mounted on a removable bracket 213 that may be plugged on the cement pipe 17, and then removed when the pipe 17 is disposed at the end of the intervention.

## LIST OF THE CITED REFERENCES

[1] A. Gangi, S. Guth, J. Imbert, H. Marin, and J.-L. Dietemann, "Percutaneous vertebroplasty: indications, technique, and results." Radiographics, vol. 23, March 2003.

[2] US 2013/0190680 of Baroud: US patent application filed on March 8, 2013 by the SOCPRA S.E.C and published on July 25, 2013.

[3]US 2009/062808 of Wolf: US patent application filed on March 2008 by Wolf (as inventor and applicant) and claiming the priority of a provisional application dated September 5, 2007, and published on March 5, 2009.

[4]US 8,523,871 of Truckai et al.: US granted patent filed on April 3, 2008 by Truckai et al. (as inventors and applicants) and claiming the priority of four provisional applications dated April 3, 2007, and granted on October 9, 2008.

**Claims**

1. Method for a dynamic control of the viscosity of an orthopedic bone cement during its curing by acting on the bone cement temperature in percutaneous vertebroplasty, within an injection device comprising a syringe, a percutaneous needle connected to the syringe via a pipe, comprising an active heat exchanger, said method being **characterized in that** it comprises the following steps:

   A. defining the time $t_o$, time at which the radiologist starts the mixing process of the bone cement;
   B. filling the syringe with the prepared bone cement;
   C. defining for said bone cement a target viscosity $\eta^*$ to be reached or maintained, said target viscosity $\eta^*$ being comprised in the range [$\eta_{min}$ -$\eta_{max}$], $\eta_{min}$ being the minimal threshold viscosity of the cement which has to be reached for beginning the injection and $\eta_{max}$ being the maximum threshold viscosity of the cement above which the injection is not possible anymore;
   D. beginning the injection of the bone cement into the vertebra;
   E. at instant t during the injection:

      e1) measuring the effective temperature T of the bone cement at the outlet of the active heat exchanger and, possibly, measuring the effective temperature $T_i$ of the bone cement at its inlet;
      e2) computing the pressure drop $\Delta P=P_o-P_i$ along the pipe between the outlet of the syringe and a given intermediate point, $P_o$ being the pressure measured at the outlet of the syringe and $P_i$ being the pressure measured at the given intermediate point on the pipe, the length between those two points being denoted as $L_{sensor}$;
      e3) computing the flow rate Q of the bone cement in the pipe;
      e4) computing the shear rate $\dot{\gamma}_p$ at the wall of the pipe as a function of the flow rate Q, the cross-section dimensions of the pipe and the intrinsic physical parameters of the cement;
      e5) calculating the instant viscosity $\eta(t)$:

         - if Q is nonzero, as a function of time t, temperature T, flow rate Q, pressure drop $\Delta P$ and shear rate $\dot{\gamma}_p$;
         - if Q has a zero value, as a function of time t and temperature T;

      e6) computing a set point temperature $T^*(t)$ associated to the target viscosity $\eta^*$ and the effective viscosity $\eta$, $\eta^*$ being function of the flow rate Q and the time t;
      e7) calculating the difference $\varepsilon_T$ between the previously determined set point temperature $T^*(t)$ and the effective temperature at the outlet of the heat exchanger T;
      e8) controlling the cooling or the heating of the bone cement throughout the control of the active heat exchanger as a function of $\varepsilon_T$;

   F. at instant t+$\Delta$t, redefining possibly the set point viscosity $\eta^*$ in the range [$\eta_{min}$; $\eta_{max}$] and repeating step E until the end of the injection, unless $\eta(t)$ has reached the maximum threshold viscosity $\eta_{max}$.

2. Method according to claim 1, wherein the step e2) of computing the pressure drop $\Delta P$ is realized between the outlet of the syringe and the outlet of the needle.

3. Method according to claim 1, wherein the step e2) of computing the pressure drop $\Delta P$ is realized between the outlet of the syringe and the outlet of the active heat exchanger.

4. Method according to claim 1, wherein the instant viscosity $\eta(t)$, if the flow rate is nonzero, is calculated according to modified Power Law as defined by formula (2) in the case of a pipe having a cylindrical geometry of radius r:

$$(2) \quad \eta(t, T, \dot{\gamma}_P) = a_{T_0}(T)K(t)\left(a_{T_0}(T)\dot{\gamma}_P\right)^{n(t)-1}$$

with

$$a_{T_0}(T) = \exp\left(\frac{-E_a}{R}\left(\frac{1}{T} - \frac{1}{T_0}\right)\right)$$

with:

- $E_a$ being the activation energy in $J.mol^{-1}$,
- T being the effective temperature of the bone cement at the outlet of the active heat exchanger,
- To being a reference temperature at which the viscosity $\eta_0$ is known,
- R being the gas constant,
- n(t) being the flow index of the bone cement at the current time t, n is either a known constant or defined as a function of to and t.
- $\dot{\gamma}_p$ being the shear rate at the wall of the pipe being given by formula (3):

$$(3)$$

$$\dot{\gamma}_P = \frac{Q}{\pi r^3} \frac{3n(t) + 1}{n(t)}$$

with r being the radius of the pipe.

- K(t) being given by formula (4):

$$(4)$$

$$Q = \left(\frac{\Delta P}{L_{sensor}}\right)^{1/n(t)} \left(\frac{r}{2K(t)}\right)^{1/n(t)} \left(\frac{\pi n(t)r^3}{3n(t) + 1}\right)$$

**5.** Method according to claim 1 to 3, wherein the instant viscosity $\eta(t)$ is calculated according to the differential equation (5):

$$(5)$$

$$\dot{\eta}(t, T, \dot{\gamma}_P) = f(\eta(t, T, \dot{\gamma}_P))$$

wherein the time derivative $\dot{\eta}$ of the viscosity is defined as a function the viscosity $\eta$.

**6.** Method according to claim 1 to 5, wherein the set point temperature T*(t) is calculated via

$$\underset{T}{\operatorname{argmin}} \dot{\eta}$$

**7.** Method according to any one of claims 1 to 5, wherein the step of measuring the flow rate Q of the bone cement in the pipe comprises a step of measuring a moving speed $V_{pist}$ of the piston of the syringe, said piston being driven to vary the volume of the cement in the syringe, the volumetric flow Q being then given by $Q = V_{pist} \cdot \pi \cdot r^2$.

**8.** Method according to any one of claims 1 to 6, wherein the controlling e8) of the active heat exchanger realizes the cooling or heating of the bone cement as a function of $\varepsilon_T$ throughout a temperature regulation scheme composed of two nested closed loops, where:

- a temperature controller $C_T$ uses the difference $\varepsilon_T$ between the previously determined set point temperature T*(t) and the effective temperature T to compute the current reference I* of the active heat exchanger, the current reference I* being limited by a current saturation block,
- a current controller $C_I$ uses the difference $\varepsilon_I$ between the current reference I* and the effective input current I to compute the input voltage U of a power supply H driving the active heat exchanger.

**9.** Method according to any one of claims 3 to 7, wherein the intravertebral pressure $P_{vertebra}$ is computed according to formula (1):

(1)

$$P_{vertebra} = P_o \left( 1 - \frac{L_{vertebra}}{L_{sensor}} \right) + \frac{L_{vertebra}}{L_{sensor}} P_i$$

with:

- $L_{vertebra}$ being the length comprised between the outlet of the syringe and the outlet of the needle.

$\eta^*$ $+$ $-$ | Temperature setpoint generation | $T^*$ | Thermal Loop | $T$ | Power Law Differential Equation | $\eta$

Viscosity loop

FIG. 1

$T^*$ $+$ $-$ $C_T$ | Current Saturation | $I^*$ $+$ $-$ $C_I$ $U$ $H$ $I$ | Heat Exchanger | $T$

Current loop

FIG. 2

FIG. 3

Force signal

Position signal

FIG. 4

FIG. 5

FIG. 6A

FIG. 6B

FIG. 7

FIG. 8

FIG. 9

FIG. 10

FIG. 11

FIG. 12

FIG. 13

FIG 14

Europäisches
Patentamt

European
Patent Office

Office européen
des brevets

# EUROPEAN SEARCH REPORT

Application Number

EP 15 30 5975

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | US 8 523 871 B2 (TRUCKAI CSABA [US] ET AL) 3 September 2013 (2013-09-03) * column 1, line 27 - line 31 * * column 4, line 40 - line 44 * * column 6, line 36 - line 41 * * column 6, line 47 - line 49 * * column 12, line 36 - line 39 * * column 12, line 59 - line 63 * * column 14, line 64 - column 15, line 2 * * column 21, line 13 - line 28 * ----- | 1 | INV. A61B17/56 |
| A | US 2008/269761 A1 (TRUCKAI CSABA [US] ET AL) 30 October 2008 (2008-10-30) * paragraphs [0061], [0063], [0080], [0082] - [0085], [0087] - [0088], [0090] * ----- | 1 | |
| A | US 2010/249793 A1 (TRUCKAI CSABA [US] ET AL) 30 September 2010 (2010-09-30) * paragraphs [0066], [0082], [0087], [0090], [0093], [0098], [0116], [0122]; figures 7,8A,9 * ----- | 1 | |
| A | WO 2007/028120 A2 (DFINE INC [US]; TRUCKAI CSABA [US]; SHADDUCK JOHN [US]) 8 March 2007 (2007-03-08) * paragraphs [0118], [0145], [0146], [0148], [0149]; claim 17 * ----- | 1 | |
| A | WO 2007/148336 A2 (DISC O TECH MEDICAL TECH LTD [IL]; GLOBERMAN OREN [IL]; BEYAR MORDECHA) 27 December 2007 (2007-12-27) * page 5, line 17 - line 21 * * page 17, line 12 - line 20 * ----- | 1 | |
| A | US 2008/300540 A1 (LEWIS VAN L [US]) 4 December 2008 (2008-12-04) * paragraphs [0034], [0037], [0040] * ----- | 1 | |

**TECHNICAL FIELDS SEARCHED (IPC)**

A61B
G05D

The present search report has been drawn up for all claims

| Place of search | Date of completion of the search | Examiner |
|---|---|---|
| Berlin | 1 December 2015 | Nice, Philip |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

# EP 3 108 833 A1

## ANNEX TO THE EUROPEAN SEARCH REPORT
## ON EUROPEAN PATENT APPLICATION NO.

EP 15 30 5975

This annex lists the patent family members relating to the patent documents cited in  the above-mentioned European search report.
The members are as contained in the European Patent Office EDP file on
The European Patent Office is in no way liable for these particulars which are merely given for the purpose of information.

01-12-2015

| Patent document cited in search report | | Publication date | Patent family member(s) | | Publication date |
|---|---|---|---|---|---|
| US 8523871 | B2 | 03-09-2013 | EP | 2155084 A1 | 24-02-2010 |
| | | | ES | 2438999 T3 | 21-01-2014 |
| | | | JP | 5174887 B2 | 03-04-2013 |
| | | | JP | 2010523225 A | 15-07-2010 |
| | | | US | 2008249530 A1 | 09-10-2008 |
| | | | US | 2008255570 A1 | 16-10-2008 |
| | | | US | 2008255571 A1 | 16-10-2008 |
| | | | WO | 2008124533 A1 | 16-10-2008 |
| US 2008269761 | A1 | 30-10-2008 | US | 2008269761 A1 | 30-10-2008 |
| | | | US | 2013226142 A1 | 29-08-2013 |
| | | | US | 2014303634 A1 | 09-10-2014 |
| | | | WO | 2008137428 A2 | 13-11-2008 |
| US 2010249793 | A1 | 30-09-2010 | NONE | | |
| WO 2007028120 | A2 | 08-03-2007 | US | 2007118144 A1 | 24-05-2007 |
| | | | US | 2007162043 A1 | 12-07-2007 |
| | | | US | 2007191858 A1 | 16-08-2007 |
| | | | US | 2007233148 A1 | 04-10-2007 |
| | | | US | 2009012525 A1 | 08-01-2009 |
| | | | US | 2012239049 A1 | 20-09-2012 |
| | | | WO | 2007028120 A2 | 08-03-2007 |
| WO 2007148336 | A2 | 27-12-2007 | ES | 2543346 T3 | 18-08-2015 |
| | | | US | 2014148866 A1 | 29-05-2014 |
| | | | WO | 2007148336 A2 | 27-12-2007 |
| US 2008300540 | A1 | 04-12-2008 | US | 2008300540 A1 | 04-12-2008 |
| | | | WO | 2008153789 A1 | 18-12-2008 |

EPO FORM P0459

For more details about this annex : see Official Journal of the European Patent Office, No. 12/82

23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8523871 B
- US 20130190680 A
- US 20090062808 A, Wolf

**Non-patent literature cited in the description**

- **A. GANGI ; S. GUTH ; J. IMBERT ; H. MARIN ; J.-L. DIETEMANN.** Percutaneous vertebroplasty: indications, technique, and results. *Radiographics,* March 2003, vol. 23